# EUROPÄISCHE PATENTSCHRIFT

(11) **EP 1 036 569 B1**
(45) Veröffentlichungstag und Bekanntmachung des Hinweises auf die Patenterteilung: **07.09.2005**
(21) Anmeldenummer: 00105665.4
(22) Anmeldetag: 17.03.2000
(51) Int. Cl.: A61M 15/00

(54) **Verfahren und Vorrichtung zur Bereitstellung einer konstanten Medikamenten-Dosis für eine inhalatorische Applikation mit niedrigem Inhalationsfluss**
Method and device for preparing a constant dose of medication administered by inhaltaion with low flow
Procédé et dispositif de formation d'une dose constante de médicament pour l'administration par inhalation avec faible débit

(30) Priorität: 18.03.1999 DE 19912265
(43) Veröffentlichungstag der Anmeldung: 20.09.2000
(73) Patentinhaber: GSF - Forschungszentrum für Umwelt und Gesundheit GmbH, 85764 Oberschleissheim (DE)
(72) Erfinder: Scheuch, Gerhard, 35285 Gemünden (DE); Sommerer, Knut, 81241 München (DE)
(74) Vertreter: Reinhard - Skuhra - Weise & Partner

(56) Entgegenhaltungen:
- EP-A- 0 965 355
- GB-A- 2 182 249

## Beschreibung

Die Erfindung betrifft ein Verfahren zur Bereitstellung einer konstanten Medikamenten-Dosis für eine inhalatorische Applikation mit niedrigem Inhalationsfluß sowie eine hierzu geeignete Vorrichtung.

Das Inhalieren von Medikamenten nimmt immer mehr an Bedeutung zu. Dabei ist zum einen zu beachten, daß Aerosolteilchen effektiv in die Lunge nur bei langsamer Inhalation, d.h. einem niedrigen Inhalationsfluß, gelangen. Bei den bisher bekannten sogenannten Pulverinhalatoren werden jedoch relativ hohe inhalatorische Flüsse in der Größenordnung von ca. 60 l/m benötigt, um dem Pulver die nötige Energie für das Entstehen inhalierbarer Teilchen durch Desagglomeration zuzuführen. Dieses Aerosolisieren von Pulver ist jedoch für die meisten Patienten nicht oder nur unzureichend möglich, da diese nicht mit einem derart hohen Fluß inhalieren können. Hinzu kommt ferner, daß für die inhalatorische Applikation von Medikamenten ein hoher Aerosolfluß ungünstig ist, da dabei ein großer Teil im Mund/-Rachenraum, insbesondere im Bereich der Stimmritze, abgeschieden wird und nicht in die Lunge gelangt.

Aus der nachveröffentlichten EP 0 965 355 A2 ist ein gesteuerter Inhalator bekannt, der zur Kontrolle des zu applizierenden Gesamtvolumens und des Atemzugvolumens ein geschlossenes Behältnis aufweist, das in einem geschlossenen Gehäuse angeordnet ist, welches ein Einwegeventil für den Austritt von Luft aus dem Inneren des Gehäuses, ein Mundstück, das mit dem Behältnis verbunden ist sowie ein die Einströmung von Luft in das Gehäuse begrenzende Düse bzw. ein Einstellventil aufweist. Das Gehäuse ist weder für die Befüllung noch für die Entnahme von Aerosol aus dem Behältnis komprimierbar und vorzugsweise aus einem durchsichtigen Material zur Füllstandskontrolle hergestellt.

Ein weiteres Problem für die inhalatorische Applikation von Medikamenten besteht darin, daß Atemzugvolumina durch die Patienten variiert werden, wodurch es auch zu einer erheblichen Variation der in der Lunge ankommenden Medikamenten-Dosis kommt. Eine derartige Variation des Atemzugvolumens tritt nicht nur bei einem Patienten auf, sondern ändert sich zudem auch von Patient zu Patient.

Demgemäß besteht ein starkes Bedürfnis nach Bereitstellung einer konstanten Medikamenten-Dosis für eine inhalatorische Applikation mit niedrigem Inhalationsfluß, um für die zu applizierenden Medikamente eine gezielte Wirkung nach Applikationsort und Applikationsdosis zu erreichen.

Gemäß der Erfindung wird dieses Bedürfnis durch ein Verfahren der eingangs genannten Art gestillt, das aus den in Patentanspruch 1 genannten Verfahrensschritten besteht. Vorrichtungsseitig ist eine Lösung durch die im Patentanspruch 5 angegebenen Merkmale vorgesehen.

Bevorzugte weitere Ausgestaltungen des Verfahrens bzw. der Vorrichtung sind den jeweils nachgeordneten Patentansprüchen zu entnehmen.

Gemäß einer bevorzugten Ausgestaltung der Erfindung wird der Saugdruck in dem Gehäuse durch externe, an diesem angreifende Kräfte, vorzugsweise durch wenigstens eine an diesem angreifende Expansionsfeder erzeugt. Alternativ kann der Saugdruck in dem Gehäuse auch durch dessen materialbedingte Rückverformungselastizität erzeugt werden, die das Gehäuse in seinen unkomprimierten Ausgangszustand zurückführt.

Durch das erfindungsgemäße Verfahren kann mit einfachen Mitteln ohne zusätzliche elektronische Steuerung eine zuverlässige Bereitstellung einer vorbestimmten konstanten Medikamenten-Dosis für eine inhalatorische Applikation erreicht werden, wobei beim Inhalieren selbst das vorgesehene luftströmungsgesteuerte Zusammendrücken zur Erzielung einer effektiven langsamen Inhalation beiträgt.

Vorrichtungsseitig ist nach der Erfindung eine Ausgestaltung nach Patentanspruch 5 vorgesehen.

Aufgrund der erfindungsgemäßen Gestaltung wird eine außerordentlich einfach herstellbare Vorrichtung verfügbar gemacht, die nach separater Aerosolerzeugung für eine Inhalation mit kleinem oder niedrigem Aerosolfluß geeignet ist.

Die Einrichtung zur Steuerung des Ein- und Auslasses von Luft weist zwei Ein-Wegventile auf, die vorzugsweise an verschiedenen Stellen mit zueinander entgegengesetzter Wirkung angebracht sind.

Das Gehäuse besitzt nach einer bevorzugten Ausgestaltung der Erfindung eine separate Einrichtung, durch die es expandierbar ist, wobei die separate Einrichtung bevorzugt mit wenigstens einer extern angreifenden Druckfeder gebildet ist und das Gehäuse annähernd zylinderförmig mit randseitigen Faltabschnitten ausgestaltet ist.

Alternativ kann statt der separaten Einrichtung oder ergänzend zu dieser das Gehäuse auch aus einem elastischen Material bestehen, das sich in den Bereitstellungszustand rückverformt.

Der Innenbehälter in dem Gehäuse besteht bevorzugt aus einem elastischen Material und ist vorzugsweise ballonförmig gebildet. Er kann nach einer Weiterbildung der Erfindung außer im Bereich des Mundstücks noch in einem davon beabstandeten Bereich an der Innenseite des Gehäuses befestigt sein, um sein Aufblähen zwecks Aerosolaufnahme zu fördern.

Gemäß einer weiteren vorteilhaften Ausgestaltung der Erfindung ist das Gehäuse wenigstens teilweise zur Füllkontrolle des Behälters durchsichtig. Hierdurch kann der die Vorrichtung benutzende Patient visuell feststellen, wie groß die Füllung ist und wieviel beim Einatmen noch in dem Behälter an Aerosol vorhanden ist.

Gemäß einer weiteren Ausgestaltung der Erfindung besitzt das Gehäuse eine lösbare Einrichtung zur Sicherung der volumenkomprimierten Stellung, d.h. der Stellung, in der das Gehäuse und der darin befindliche Innenbehälter weitestgehend in Richtung auf das Mundstück zusammengezogen sind. Diese sogenannte volumenkomprimierte Stellung läßt sich durch die lösbare Einrichtung, beispielsweise in Form einer Spanneinrichtung, auch für einen Konfektionierzustand der Vorrichtung zu Verkaufszwecken nutzen.

Gemäß der erfindungsgemäßen Vorrichtung wird somit ein unter Spannung stehendes Gehäuse, das zumindest teilweise durchsichtig ist, mit einem darin befindlichen ballonförmigen Innenbehälter verfügbar gemacht. An dem Mundstück kann ein beliebiges System zur Pulver-Aerosol-Erzeugung (DPI: Dry Powder Inhalator) luftdicht in einfacher Weise angebracht werden. Wenn das Gehäuse aus seiner volumenkomprimierten Stellung zur vorgesehenen, automatisch ablaufenden Expansion freigegeben wird, entfaltet sich ebenfalls der in dem Gehäuse befindliche Innenbehälter durch den entstehenden Unterdruck, wobei durch den Pulver-Aerosol-Inhalator Luft in den ballonförmigen Innenbehälter gesogen und damit das Aerosol in der gewünschten Dosis erzeugt wird. Dabei kann nach einer bevorzugten weiteren Ausgestaltung der Erfindung im Mundstück ein sogenannter Impaktionsabscheider angeordnet sein, der größere Aerosolteilchen mit über 10 µm aerodynamischem Durchmesser zurückhält, da diese sowieso nicht inhalierbar sind.

Nach erfolgter Bereitstellung befindet sich in dem ballonförmigen Innenbehälter stets eine konstante Menge eines vorbestimmbaren Aerosols, die nur durch den Pulver-Aerosol-Inhalator, die luftströmungsgesteuerte Expansion des Gehäuses und die Konstruktion des Impaktionsabscheiders bestimmt ist. Die Handhabung ist auch für ungeübte Personen außerordentlich einfach, und nach der beschriebenen Bereitstellung braucht ein Patient nur noch den Pulver-Aerosol-Inhalator vom Mundstück zu nehmen, sein Lippen an das Mundstück setzen und den ballonförmigen Innenbehälter mit einem Atemzug zu entleeren. Dabei ist durch die Luftströmungssteuerung für den Bereich zwischen Gehäuse und Innenbehälter, der durch ein Einwegventil verwirklicht ist, sichergestellt, daß der Patient nur mit einem maximal einstellbaren Fluß einatmen kann, und durch diese Flußreduzierung und durch die Begrenzung des Volumens des ballonförmigen Innenbehälter atmet ein Patient immer die gleiche Menge Aerosol ein. Dabei wird vorteilhaft eine Deponierung im extrathorakalen Bereich minimiert, da die größeren Teilchen schon im System hängenbleiben und der Fluß begrenzt ist.

Aufgrund der erfindungsgemäßen Ausgestaltung des Verfahrens und der Vorrichtung wird vorteilhaft die Voraussetzung dafür geschaffen, daß eine inhalatorische Applikation unabhängig vom möglichen maximalen Inhalationsfluß des individuellen Patienten ist, wobei kein unerwünschtes Medikament im Rachenraum wegen Vermeidung der extrathorakalen Deposition verbleibt und sichergestellt ist, daß die Dosis konstant ist, da ein Patient ein konstantes Volumen und einen nach oben begrenzten Fluß inhaliert. Die Vorrichtung läßt sich außerordentlich klein und mit günstigen Herstellungskosten bereitstellen, wobei für den Verkauf und Transport ein kompakter zusammengefalteter Zustand günstig ist. Es wird keine Elektronik und nur ein geringer Materialaufwand benötigt, und die Vorrichtung läßt sich mit allen schon zugelassenen Trockenpulverinhalatoren problemlos einsetzen.

Durch das neue Verfahren und die neue Vorrichtung wird somit vorteilhaft eine konstante Medikamenten-Dosis für eine inhalatorische Applikation mit niedrigem Inhalationsfluß verfügbar gemacht, die eine sichere und zuverlässige Volumenbemessung für einen Beaufschlagungsbereich erlaubt, während bisher bekannte Trockenpulverinhalatoren mit den eingangs beschriebenen Unzulänglichkeiten bisher nur 2-10% des medikamentösen Wirkstoffs in der richtigen Dosis einbringen konnten. Damit ist gleichzeitig mit dem erfindungsgemäßen Verfahren und der erfindungsgemäßen Vorrichtung eine deutlich verbesserte Ausnutzung der Medikamente selbst erreicht, wodurch sich auch die Kosten für die Behandlung von Patienten über einen längeren Zeitraum verringern.

Hinsichtlich des Materials der Vorrichtung ist hinsichtlich des Innenbehälters ein antistatisches medikamentenzugelassenes Material vorgesehen, während für das Gehäuse wegen der fehlenden Kontaktierung mit den Medikamenten die Materialauswahl nur durch die vorgesehene Funktion beschränkt ist.

Nachfolgend wird die Erfindung unter Bezugnahme auf ein Ausführungsbeispiel einer Vorrichtung näher erläutert. Es zeigen:
- Fig. 1: eine schematische Darstellung einer Vorrichtung im zusammengefalteten Zustand; und
- Fig. 2: eine schematische Darstellung der Vorrichtung gemäß Fig. 1 nach deren Entfaltung in einem Zustand, in dem eine konstante Medikamenten-Dosis für eine inhalatorische Applikation mit niedrigem Inhalationsfluß vorliegt.

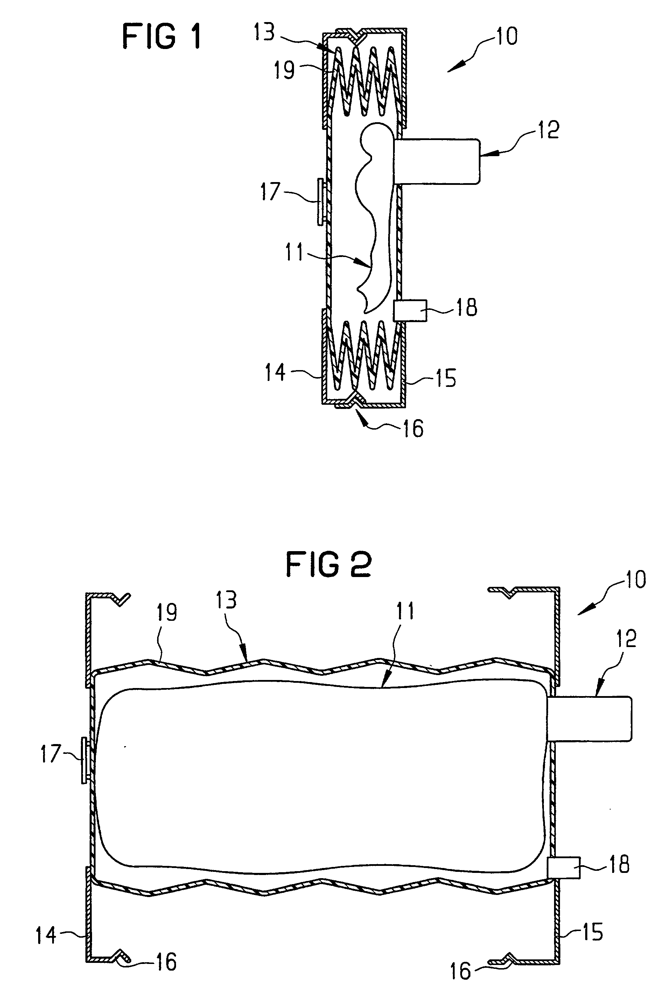

In den Fig. 1 und 2 ist eine Vorrichtung 10 zur Bereitstellung einer konstanten Medikamenten-Dosis für eine inhalatorische Applikation mit niedrigem Inhalationsfluß schematisiert dargestellt. Die Vorrichtung 10 besteht aus einem volumenreduzierbaren geschlossenen Innenbehälter 11 in Form eines elastischen Ballons, der mit einem Mundstück 12 verbunden ist. Der Innenbehälter 11 ist von einem volumenreduzierbaren Gehäuse 13 geschlossen umgeben, aus dem das Mundstück 12 abgedichtet herausgeführt ist. Mit 14 und 15 ist eine zweiteilige Spannvorrichtung mit wechselseitiger Rasteinformung 16 gezeigt, die an gegenüberliegenden Seitenabschnitten des Gehäuses 13 befestigt ist und das Gehäuse 13 in dem in Fig. 1 dargestellten volumenreduzierten Zustand hält. Mit 18 ist schematisiert ein Einwegventil angedeutet, das dafür sorgt, daß Luft aus dem Bereich zwischen dem Innenbehälter 11 und dem Gehäuse 13 beim Expandieren des Innenbehälters 11 austreten kann, nicht jedoch durch dieses Ventil einströmt. Das Ventil 18 ist dabei hinsichtlich seiner geometrischen Gestaltung so ausgelegt, daß es bei dem Expandieren des Innerbehalters 11 für eine nachfolgend noch erläuterte Befüllung desselben mit gleichbleibendem Fluß zur vorgesehenen Aerosolerzeugung sorgt. Das Ventil 17 ist ebenfalls als Einwegventil ausgestaltet und sorgt dafür, daß Luft in den Raum zwischen dem Gehäuse 13 und dem Innenbehälter 11 beim Inhalieren eintritt, durch das Ventil 17 jedoch nicht austritt. Das Ventil 17 ist dabei für die vorgesehene Begrenzung des Inhalationsflusses aufgrund seiner geometrischen Gestaltung vorgesehen, wobei in nicht dargestellter Weise auch eine Einstellbarkeit des Ventils 17 vorgesehen sein kann. Die Ventile 18 und 17 sorgen somit für ein luftströmungsgesteuertes Expandieren und Zusammendrücken des ballonförmigen Innenbehälters 11, um einerseits ein vorgesehenes Medikamentenaerosol mit gleichbleibendem Fluß bereitzustellen (Ventil 18) und andererseits die Voraussetzung für eine inhalatorische Applikation mit niedrigem Inhalationsfluß über das Ventil 17 zu schaffen.

Das Gehäuse 13 ist bei dem in den Fig. 1 und 2 dargestellten Ausführungsbeispiel annähernd zylinderförmig oder rechteckig mit randseitigen Faltabschnitten 19 gebildet. Für die vorgesehene Expandierung des Gehäuses 13 besteht dieses aus einem elastischen Material, das bestrebt ist, die in Fig. 2 gezeigte Expansionsstellung rückverformend einzunehmen. Alternativ kann in nicht dargestellter Weise auch wenigstens eine extern angreifende Druckfeder für die gewünschte Expansion gemäß Fig. 2 vorgesehen sein.

Fig. 2 zeigt die Vorrichtung gemäß Fig. 1 in einem expandierten Zustand des Gehäuses 13 und einem expandierten, befüllten ballonförmigen Innenbehälter 11. Die Vorrichtung 10 hat diesen Zustand nach Lösen der Spanneinrichtung 14-16 selbsttätig eingenommen. In der in Fig. 2 gezeigten Stellung ist die Vorrichtung bereit zur vorgesehenen inhalatorischen Applikation einer vorgesehenen bestimmten Medikamenten-Dosis mit niedrigem Inhalationsfluß.

Für die Erzeugung dieser vorgesehenen Medikamenten-Dosis in Aerosolform ist dabei vorgesehen, daß an das Mundstück 12 ein herkömmlicher Pulver-Aerosol-Inhalator in der Stellung der Vorrichtung 10 gemäß Fig. 1 abgedichtet aufgesetzt wird. Nach Lösen der Spanneinrichtung 14-16 expandiert das Gehäuse 13, und durch den Unterdruck entfaltet sich ebenfalls der ballonförmige Innenbehälter 11, wobei durch den Pulver-Aerosol-Erzeuger Luft zur Aerosolerzeugung eingesogen wird und diese Befüllung des ballonförmigen Innenbehälter 11 aufgrund der Luftströmungssteuerfunktion des Ventils 18 mit gleichbleibendem hohen Fluß erfolgt, beispielsweise in der Größenordnung von 60 l/min, um dem Pulver des Inhalators die nötige Energie zuzuführen, damit die "inhalierbaren Teilchen" durch Desagglomeration entstehen können.

Nach Erreichen des in Fig. 2 dargestellten Zustandes braucht ein Patient nur noch den zuvor an das Mundstück abgedichtet angesetzten Pulver-Aerosol-Erzeuger abzunehmen und kann dann die nach Volumen und Medikamenten-Dosis bereitgestellte Aerosolbefüllung mit einem Atemzug in gewünschter Weise in der Lunge applizieren, wobei aufgrund des Ventils 17 das Einatmen mit gleichbleibendem niedrigen Fluß sichergestellt ist.

Die erfindungsgemäß bereitstellbaren zu applizierenden Substanzen schließen Wirkstoffe, Medikamente, Verbindungen, Zusammensetzungen oder Mischungen von Stoffen ein, die einen pharmakologischen, oft vorteilhaften Effekt erzielen. Dies schließt Speisen, Nahrungsergänzungsstoffe, Nährstoffe, Medikamente, Impfstoffe, Vitamine und weitere nützliche Wirkstoffe mit ein. Die Substanzen schließen weiterhin alle physiologisch oder pharmakologisch aktiven Substanzen ein, die einen lokalen oder systemischen Effekt in einem Patienten bewirken. Der aktive Wirkstoff, der zugeführt werden kann, schließt Antikörper, antivirale Wirkstoffe, Antiepileptika, Analgetika, entzündungshemmende Wirkstoffe und Bronchodilatatoren ein und kann eine organische oder anorganische Verbindung sein, die ohne Beschränkungen auch Medikamente einschließt, die auf das periphere Nervensystem, adrenerge Rezeptoren, cholinerge Rezeptoren, Skelettmuskulatur, kardiovaskuläres System, glatte Muskulatur, Blutkreislaufsystem, neuronale Verbindungen, endokrines- und Hormonsystem, Immunsystem, reproduktives System, Skelettsystem, Nahrungszufuhr- und exkretorisches System, Histaminkaskade oder zentrales Nervensystem wirken. Geeignete Wirkstoffe können z. B. aus Polysacchariden, Steroiden, Hypnotika und Sedativa, antriebssteigernden Mitteln, Beruhigungsmitteln, krampflösenden und muskelentspannenden Mitteln, Antiparkinson-Substanzen, Analgetica, Entzündungshemmern, antimikrobiellen Wirkstoffen, Antimalariamitteln, Hormonen inklusive empfängisverhütenden Mitteln, Symphaticomimetica, Polypeptiden und Proteinen, die physiologische Effekte hervorrufen, Diuretica, Fettstoffwechsel regulierenden Substanzen, antiandrogenen Wirkstoffen, gegen Parasiten gerichtete Mitteln, neoplastisch und antineoplastisch wirkenden Stoffen, Antidiabetika, Nahrungs- und Nahrungsergänzungsstoffen, wachstumsfördernden Stoffen, Fetten, stuhlregulierenden Stoffen, Elektrolyten, Impfstoffen und Diagnostika bestehen.

Das erfindungsgemäße Verfahren eignet sich besonders zur Bereitstellung der nachfolgenden Wirkstoffe zur inhalativen Applikation , ist aber nicht darauf beschränkt:
Insulin, Calcitonin, Erythropoietin (EPO), Faktor VIII, Faktor IX, Cyclosporin, Granulozyte Colony Stimulating Factor (GCSF), Alpha-1 Proteinase Inhibitor, Elcatonin, Granulocyte Makrophage Colony Stimulating Factor (GMCSF), Wachstumshormone, menschliches Wachstumshormon (HGH), Growth hormone releasing hormone (GHRH), Heparin, niedermolekulares Heparin (LMWH), Interferon alpha, Interferon beta, Interferon gamma, Interleukin-2, Luteinizing hormone releasing hormone (LHRH), Somatostatin, Somatostatin-Analoge einschließlich Octreotide, Vasopressinanaloge , Follikel stimulierendes Hormon (FSH), Insulin like growth factor, Insulintropin, Interleukin-1 Rezeptorantagonist, Interleukin-3, Interleukin-4, Interleukin-6, Macrophage colony stimulating Factor (M-CSF), Nerven-Wachstumsfaktor, Parathyroidhormon (PTH), Thymosin alpha 1, IIb/-IIIa Inhibitor, Alpha-1 Antitrypsin, Antikörper gegen respiratorisch syncytischen Virus, Cystic fibrosis transmembrane regulator gene (CFTR), Desoxyribonuclease (Dnase), Bactericide, permeability increasing protein (BPI), anti-CMV Antikörper, Interleukin-1 Rezeptor, Retinol-Säuren, Pentamidine, Albuterolsulfat, Metaproterenolsulfat, Beclomethasondiprepionat, Triamcinolonacetamid, Budesonidacetonide, Ipratropiumbromid, Flunisolide, Fluticasone, Cromolynsodium, Ergotamintartrat und die Analogen, Agonisten und Antagonisten der oben genannten Stoffe. Aktive Wirkstoffe können weiterhin Nukleinsäuren in Form von reinen Nukleinsäuremolekülen, viralen Vektoren, assoziierten viralen Partikeln, Nukleinsäuren, die mit Lipiden oder einem Lipide beinhaltenden Material assoziiert oder darin enthalten sind, Plasmid-DNA oder -RNA oder andere Konstrukte aus Nukleinsäuren sein, die geeignet sind für die Zell-Transfection oder -Transformation, besonders bei Zellen der alveolären Region der Lunge. Der aktive Wirkstoff kann in verschiedenen Formen vorliegen, wie lösliche oder unlösliche geladene oder ungeladene Moleküle, Komponenten stoffe. Der aktive Wirkstoff kann bestehen aus natürlich vorkommenden Molekülen, oder deren rekombinanter Produktion, oder die Moleküle können Analoge der natürlich vorkommenden oder rekombinant erzeugten aktiven Wirkstoffe sein, bei denen eine oder mehrere Aminosäuren addiert oder entfernt wurden. Weiterhin kann der aktive Wirkstoff abgeschwächten Lebendimpfstoff oder abgetötete Viren für den Impfstoffgebrauch enthalten. Im Falle des Wirkstoffes Insulin sind natürlich extrahiertes menschliches Insulin, rekombinantes menschliches Insulin, aus Rindern und/oder Schweinen extrahiertes Insulin, rekombinantes Schweine- oder Rinderinsulin und Mischungen der oben genannten Insuline eingeschlossen. Das Insulin kann in reiner, also in substanziell gereinigter Form vorliegen, kann aber auch Auszüge wie kommerziell üblich enthalten. Im Ausdruck "Insulin" sind auch Insulin-Analoge enthalten, bei denen eine oder mehrere der Aminosäuren des natürlich vorkommenden oder rekombinanten Insulins addiert oder entfernt wurden.

## Patentansprüche

1. Verfahren zur Bereitstellung einer vorbestimmten konstanten Medikamenten-Dosis für eine inhalatorische Applikation mit niedrigem Inhalationsfluß, bestehend aus den folgenden Verfahrensschritten:
Vorsehen eines volumenreduzierbaren geschlossenen Innenbehälters (11) mit Mundstück (12) in einem geschlossenen komprimierbaren Gehäuse (13), wobei der Innenbehälter (11) zur Aufnahme eines vorbestimmten, vorzugsweise mit einem Atemzug zu inhalierenden Volumens einer Medikamenten-Aerosol-Menge vorgesehen ist, und wobei das Gehäuse (13) zum Expandieren des Innenbehälters (11) aus einem komprimierten Zustand ein Luftauslaßventil (18) mit hohem Durchfluß für Luft aus dem Bereich zwischen Gehäuse (13) und Innenbehälter(11) und ein Lufteinlaßventil (17) für die Steuerung des Entleerens des Innenbehälters (11) durch das aus dem Gehäuse (13) geführte Mundstück (12) mit einem vergleichsweise niedrigeren Durchfluß aufweist;
Zusammendrücken des Gehäuses (13) in Richtung des Mundstücks (12) unter Steuerung der Luftauslaßströmung durch das Luftauslaßventil (18);
Anschließen einer Einrichtung zur Pulver-Aerosol-Erzeugung an das Mundstück (12); und
luftströmungsgesteuertes Expandieren des Innenbehälters (11) durch Aufbringen eines Saugdrucks in dem Gehäuse (13) zum Einführen von Luft durch die Pulver-Aerosol-Erzeugungseinrichtung zur vorgesehenen Aerosol-Befüllung des Innenbehälters (11).

2. Verfahren nach Anspruch 1,
**dadurch gekennzeichnet,**
**daß** der Saugdruck in dem Gehäuse (13) durch extern an dieses angreifende Kräfte erzeugt wird.

3. Verfahren nach Anspruch 1,
**dadurch gekennzeichnet,**
**daß** der Saugdruck in dem Gehäuse (13) durch wenigstens eine an diesem angreifende Expansionsfeder erzeugt wird.

4. Verfahren nach Anspruch 1,
**dadurch gekennzeichnet,**
**daß** der Saugdruck in dem Gehäuse (13) durch Wahl eines elastischen rückverformenden Werkstoffs für das Gehäuse (13) erzeugt wird.

5. Vorrichtung zur Bereitstellung einer vorbestimmten konstanten Medikamenten-Dosis für eine inhalatorische Applikation mit niedrigem Inhalationsfluß, bestehend aus einem volumenreduzierbaren geschlossenen Innenbehälter (11);
einem mit dem Innenbehälter (11) verbundenen Mundstück (12), an dem zur Aerosol-Bereitstellung ein Pulver-Aerosol-Inhalator anschließbar ist;
einem den Innenbehälter (11) geschlossen umgebenden, volumenreduzierbaren Gehäuse (13), aus dem das Mundstück (12) abgedichtet herausgeführt ist; und aus Einwegventilen (17, 18) zur Steuerung des Luftein- und Luftauslasses aus dem bzw. in den Bereich zwischen Innenbehälter (11) und Gehäuse (13);
wobei das Gehäuse (13) zur Befüllung des Innenbehälters (11) mit dem vorgesehenen Aerosol-Volumen aus einem volumenreduzierten Zustand in einen vorgesehenen expandierten Bereitstellungszustand bringbar ist, und
wobei das für den Lufteinlaß vorgesehene Einwegventil (17) für den für einen vergleichsweise niedrigeren Durchfluß als das für den Luftauslaß vorgesehene Einwegventil (18) ausgebildet ist.

6. Vorrichtung nach Anspruch 5,
**dadurch gekennzeichnet,**
**daß** das Gehäuse (13) eine separate Einrichtung aufweist, durch die es expandierbar ist.

7. Vorrichtung nach Anspruch 6,
**dadurch gekennzeichnet,**
**daß** die separate Einrichtung mit wenigstens einer extern angreifenden Druckfeder gebildet ist.

8. Vorrichtung nach einem der Ansprüche 5 bis 7,
**dadurch gekennzeichnet,**
**daß** das Gehäuse (13) annähernd zylinderförmig oder rechteckig mit randseitigen Faltabschnitten (19) gebildet ist.

9. Vorrichtung nach einem der Ansprüche 5 bis 8,
**dadurch gekennzeichnet,**
**daß** das Gehäuse (13) aus einem elastisch in den Bereitstellungszustand rückverformbaren Material besteht.

10. Vorrichtung nach einem der Ansprüche 5 bis 9,
**dadurch gekennzeichnet,**
**daß** der Innenbehälter (11) aus einem elastischen Material besteht.

11. Vorrichtung nach Anspruch 10,
**dadurch gekennzeichnet,**
**daß** der Innenbehälter (11) ballonförmig gebildet ist.

12. Vorrichtung nach einem der Ansprüche 5 bis 10,
**dadurch gekennzeichnet,**
**daß** der Innenbehälter (11) außer im Bereich des Mundstücks (12) noch in einem davon beabstandeten Bereich an der Innenseite des Gehäuses (13) befestigt ist.

13. Vorrichtung nach einem der Ansprüche 5 bis 12,
**dadurch gekennzeichnet,**
**daß** das Gehäuse (13) wenigstens teilweise zur Füllstandskontrolle des Innenbehälters (11) durchsichtig ist.

14. Vorrichtung nach einem der Ansprüche 5 bis 13,
**dadurch gekennzeichnet,**
**daß** das Gehäuse (13) eine lösbare Einrichtung (14-16) zur Sicherung der volumenreduzierten Stellung aufweist.

15. Vorrichtung nach einem der Ansprüche 5 bis 14,
**dadurch gekennzeichnet,**
**daß** in dem Mundstück (12) ein Impaktionsabscheider angeordnet ist.

## Claims

1. A method of providing a predetermined constant medicament dose for an inhalational administration at a low inhalation flow rate, comprising the following steps:
providing a closed inner container (11), which is reducible in terms of volume, with a mouthpiece (12) within a closed compressible housing (13), said inner container (11) being provided for receiving a predetermined preferably in one breath inhalable volume of a quantity of medicated aerosol, and wherein said housing (13) comprises an air outlet valve (18) for a high flow rate of air out of the space between the housing (13) and the inner container (11) for expanding the inner container (11) from a compressed condition, and an air inlet valve (17) having a comparable lower flow rate for controlling the discharge of the inner container (11) through the mouthpiece (12) which is led out of said housing (13);
compressing said housing (13) in the direction of said mouthpiece (12), controlling the air outlet flow by said air outlet valve (18);
connecting to said mouthpiece (12) a means for generating powder aerosol; and
air flow controlled expanding of said inner container (11) by applying a suction pressure in the housing (13) to introduce air through said powder aerosol generating means for the provided filling of said inner container (11) with aerosol.

2. Method according to claim 1, **characterized in that** the suction pressure in said housing (13) is generated by externally applying forces thereto.

3. Method according to claim 1, **characterized in that** the suction pressure in said housing (13) is generated by at least one expansion spring.

4. Method according to claim 1, **characterized in that** the suction pressure in said housing (13) is generated by the selection of a material for said housing (13) which returns resiliently into its original shape.

5. A device for providing a predetermined constant medicament dose for an inhalational administration at a low inhalation flow rate, comprising
a closed inner container (11) reducible in terms of volume;
a mouthpiece (12) connected to said inner container (11) and a adapted for the connection of a powder aerosol inhaler for providing an aerosol;
a housing (13) which closely encloses said inner container (11) and which is adapted to be reduced in terms of volume, said mouthpiece (12) being led out therefrom in a sealed manner; and
inlet valves (17, 18) for controlling the air inlet and air outlet out of or into the space between the inner container (11) and the housing (13);
said housing (13) being adapted to be changed from a volume-reduced condition into an envisaged expanded availability condition for filling said inner container (11) with the envisaged aerosol volume,
said inlet valve (17) which is provided for air inlet, being configured for a comparable lower flow than the inlet valve (18) provided for the air outlet.

6. Device according to claim 5, **characterized in that** said housing (13) comprises a separate means by which it is expandable.

7. Device according to claim 6, **characterized in that** said separate means is formed with at least one externally applying compression spring.

8. Device according to on of claims 5 to 7, **characterized in that** said housing (13) is formed approximately in shape of a cylinder or rectangular with folding portions (19) at its side.

9. Device according to one of claims 5-8, **characterized in that** said housing (13) consists of a material which returns resiliently into its original shape in the availability condition.

10. Means according to one of claims 5-9, **characterized in that** said inner container (11) consists of an elastic material.

11. Device according to claim 10, **characterized in that** the inner container (11) is configured to have a balloon-like shape.

12. Device according to one of claims 5-10, **characterized in that** the inner container (11) is fastened not only in the region of said mouthpiece (12) but additionally also in region spaced therefrom at the inside of said housing (13).

13. Device according to one of claims 5-12, **characterized in that** said housing (13) is at least partly transparent for a check of the filling level in said inner container (11).

14. Device according to one of claims 5-13, **characterized in that** said housing (13) comprises a releasable means (14-16) for securing the volume-reduced position.

15. Device according to one of claims 5-14, **characterized in that** an impaction separator is arranged in said mouthpiece (12).

## Revendications

1. Procédé de préparation d'une dose constante prédéterminée de médicament pour administration par inhalation à faible débit, composé des étapes suivantes :
- fourniture d'un récipient interne fermé réductible en volume (11) avec embout (12) dans un boîtier compressible fermé (13), dans lequel le récipient interne (11) est prévu pour recueillir un volume prédéterminé, à inhaler de préférence en une inspiration, d'une quantité d'aérosol de médicament et dans lequel le boîtier (13) présente, pour l'expansion du récipient interne (11) à partir d'un état comprimé, une soupape de sortie d'air (18) avec un débit élevé pour l'air issu de la zone comprise entre le boîtier (13) et le récipient interne (11) et une soupape d'entrée d'air (17) pour la commande du vidage du récipient interne (11) par l'embout acheminé du boîtier (13) avec un débit relativement plus faible;
compression du boîtier (13) en direction de l'embout (12) par commande du courant de sortie d'air par la soupape de sortie d'air (18);
raccordement d'un dispositif de production d'aérosol de poudre sur l'embout (12); et
expansion du récipient interne (11) commandée par courant d'air par application d'une pression d'aspiration dans le boîtier (13) pour introduire de l'air à travers le dispositif de production d'aérosol de poudre pour le remplissage prévu en aérosol du récipient interne (11).

2. Procédé selon la revendication 1, **caractérisé en ce que** la pression d'aspiration dans le boîtier (13) est produite par des forces qui s'appliquent extérieurement sur celui-ci.

3. Procédé selon la revendication 1, **caractérisé en ce que** la pression d'aspiration dans le boîtier (13) est produite par au moins un ressort d'expansion s'appliquant sur celui-ci.

4. Procédé selon la revendication 1, **caractérisé en ce que** la pression d'aspiration dans le boîtier (13) est produite en choisissant un matériau à rappel élastique pour le boîtier (13).

5. Dispositif de préparation d'une dose constante prédéterminée de médicament pour administration par inhalation à faible débit, composé
d'un récipient interne fermé réductible en volume (11);
d'un embout (12) relié au récipient interne (11), auquel on peut raccorder un inhalateur d'aérosol de poudre;
d'un boîtier réductible en volume fermé (13) entourant le récipient interne (11), d'où est acheminé de manière étanche l'embout (12); et
de soupapes unidirectionnelles (17, 18) pour commander l'entrée et la sortie d'air de ou dans la zone entre le récipient interne (11) et le boîtier (13);
dans lequel le boîtier (13) peut être amené, pour le remplissage du récipient interne (11) avec le volume d'aérosol prévu, d'un état de volume réduit à un état de préparation en expansion prévu, et
dans lequel la soupape unidirectionnelle (17) prévue pour l'entrée d'air est conçue pour un débit relativement plus faible que la soupape unidirectionnelle (18) prévue pour la sortie d'air.

6. Dispositif selon la revendication 5, **caractérisé en ce que** le boîtier (13) présente un dispositif séparé par lequel il peut être placé en expansion.

7. Dispositif selon la revendication 6, **caractérisé en ce que** le dispositif séparé est formé avec au moins un ressort de compression s'appliquant de l'extérieur.

8. Dispositif selon l'une quelconque des revendications 5 à 7, **caractérisé en ce que** le boîtier (13) est formé de manière approximativement cylindrique ou rectangulaire avec des sections de bord en accordéon (19).

9. Dispositif selon l'une quelconque des revendications 5 à 8, **caractérisé en ce que** le boîtier (13) est constitué d'un matériau à rappel élastique à l'état de préparation.

10. Dispositif selon l'une quelconque des revendications 5 à 9, **caractérisé en ce que** le récipient interne (11) est composé d'un matériau élastique.

11. Dispositif selon la revendication 10, **caractérisé en ce que** le récipient interne (11) est en forme de ballon.

12. Dispositif selon l'une quelconque des revendications 5 à 10, **caractérisé en ce que** le récipient interne (11) est fixé, outre à l'extérieur dans la zone de l'embout (12), également dans une zone qui en est distante sur le côté interne du boîtier (13).

13. Dispositif selon l'une quelconque des revendications 5 à 12, **caractérisé en ce que** le boîtier (13) est transparent au moins partiellement pour contrôler le niveau du récipient interne (11).

14. Dispositif selon l'une quelconque des revendications 5 à 13, **caractérisé en ce que** le boîtier (13) présente un dispositif amovible (14-16) pour établir la position de volume réduit.

15. Dispositif selon l'une quelconque des revendications 5 à 14, **caractérisé en ce qu'**un séparateur d'ions d'impact est aménagé dans l'embout (12).
